Europäisches Patentamt

(19) European Patent Office

Office européen des brevets

(11) Publication number: **0 383 697**
**A2**

(12) # EUROPEAN PATENT APPLICATION

(21) Application number: **90400448.8**

(51) Int. Cl.5: **A61B 5/0402**

(22) Date of filing: **19.02.90**

(30) Priority: **17.02.89 JP 38904/89**
**17.02.89 JP 38905/89**
**17.02.89 JP 38906/89**
**17.02.89 JP 38907/89**
**17.02.89 JP 38908/89**
**17.02.89 JP 38909/89**

(43) Date of publication of application:
**22.08.90 Bulletin 90/34**

(84) Designated Contracting States:
**AT BE CH DE DK ES FR GB GR IT LI LU NL SE**

(71) Applicant: **KABUSHIKI KAISHA GAKUSHU KENKYUSHA**
**40-5, Kami-ikedai 4 chome, Ohta-ku**
**Tokyo(JP)**

Applicant: **Musha, Toshimitsu**
**13-17, Minami-tsukushino 2-chome**
**Machida-shi, Tokyo(JP)**

(72) Inventor: **Musha, Toshimitsu**
**13-17, Minami-tsukushino 2-chome**
**Machida-shi, Tokyo(JP)**
Inventor: **Harumi, Ken-ichi**
**3-36-5, Takadanobaba, Shinjuku-ku**
**Tokyo(JP)**
Inventor: **Wei, Daming**
**632-6, Miyauchi, Nakahara-ku**
**Kawasaki-shi, Kanagawa(JP)**
Inventor: **Yamada, Goichiro**
**6-31-5, Minami-magome, Ohta-ku**
**Tokyo(JP)**

(74) Representative: **Rodhain, Claude et al**
**Cabinet Claude Rodhain 30, rue la Boétie**
**F-75008 Paris(FR)**

(54) **Electrocardiographic process simulator.**

(57) A simulator (1) simulates bioelectric phenomena of a heart based on excited conditions of cells which make up a heart model. The simulator (1) includes a heart model setting mechanism for constructing the heart model by establishing I, J, K blocks in respective vertical, horizontal, and height directions, and also by assigning cells to a desired one of the blocks, a section display mechanism (32) for visually displaying a section of a desired one of the blocks of the heart model and adjacent sections or a perpendicular section simultaneously, and an operating mechanism (20) for assigning cells and types thereof to the block whose section is displayed by the section display mechanism.

FIG.1

FIG.2

# ELECTROCARDIOGRAPHIC PROCESS SIMULATOR

The present invention relates to a simulator of electrocardiographic processes of a human heart.

Body surface potentials of a human body represent an electromotive force (EMF) distribution in the heart as it is observed on the entire surface of the human body, and can give more electrocardiographic information than the conventional electrocardiograms. However, it is impossible in principle to obtain an inverse mathematical estimation of an EMF distribution in the myocardium based on a body surface potential distribution. The presently available inverse problem solution is too direct to expect much from since measurement errors are large, the conductivity distribution in human bodies is uncertain, and other unpredictable factors such as different individual body shapes are involved.

In light of the above considerations, there have been proposed various methods for simulating the excitation and recovery process of a heart using a three-dimensional heart model. One simulation method simulates the depolarization process, whereas another simulation method deals with the repolarization process, simulating the T wave. These methods, however, have not been able to simulate any given heart diseases since they have used an experimentally measured time at which the myocardial cell excitation starts. Another problem with these prior methods is that since the components of the heart model are simplified to facilitate simulation calculations, the calculated body surface potentials cannot be compared with actually measured values. According to another simulation proposal, action potential waveforms of myocardial cells are limited to only two kinds for endocardial and epicardial cells, with the result that the pericardial potential across the boundary between the endocardial and epicardial cells is of a greatly discontinuous nature. The discontinuous nature of the pericardial potential causes electrocardiographic processes to be simulated as if an extra EMF were present.

Stated otherwise, the heart models used by the conventional electrocardiographic process simulators have not been versatile as they are designed for particular purposes. Therefore, it has been difficult to construct a three-dimensional heart model close to an actual heart. For this reason, it has been almost impossible to simulate electrocardiographic processes of a heart with respect to any desired heart diseases. Specifically, it has been impossible to determine the correlation between an abnormal heart condition and body surface potentials, and to obtain an electrocardiogram (ECG) at a desired point on the body surface.

The three-dimensional heart model comprises a number of, often several tens of thousands, small cells which are combined into a three-dimensional heart configuration. Different kinds of heart muscles such as a sinoatrial node, an atrial muscle, etc., are assigned to these cells.

The three-dimensional heart model is corrected several times by trial and error until the results of simulation processes substantially agree with clinical experimental results. When such a corrective action is taken, the simulation system which employs the above proposed heart model displays only a model section including a region to be corrected. Therefore, the relationship between the region to be corrected and surrounding regions, or the relationship between the region to be corrected and the entire heart model, cannot be perceived immediately. As a consequence, it has been difficult to effect corrective actions such as the addition of a cell, the change of a cell type, or the like.

A vectorcardiogram (VCG) which is obtained by the above simulation process is displayed such that an EMF distribution in the heart at each time in the excitation propa gation is expressed by a single typical electric dipole, and that the locus of terminal ends of the vector components of the typical electric dipole is displayed with the starting ends of the vector components being at the origin. Therefore, a VCG is displayed as a loop-like locus. While a 12-lead ECG gives more information than a VCG, the VCG can express the excitation propagation in the heart in greater detail than the 12-lead ECG. However, the VCG which has the above display mode does not allow the observer to easily recognize the specific time in the excitation propagation to which a certain point on the displayed locus corresponds.

The present invention has been devised in an effort to solve the aforesaid problems of the conventional electrocardiographic process simulators.

It is an object of the present invention to provide an electrocardiographic process simulator which can construct a three-dimensional heart model close to an actual human heart, can simulate electrocardiographic processes with respect to any desired heart diseases, i.e., can determine the correlation between an abnormal heart condition and body surface potentials, and obtain an electrocardiogram at a desired point on the body surface, and also allows the constructed three-dimensional to be easily corrected.

Another object of the present invention is to provide an electrocardiographic process simulator which permits an easy understanding of a correlation with respect to time

According to the present invention, there is provided an electrocardiographic process simulator for constructing a heart model of a cluster of cells and obtaining bioelectric phenomena of the heart model

2

from the excited conditions of the cells which are determined by applying predetermined rules based on bioelectric characteristics of heart cells to the cells of the heart model, the electrocardiographic process simulator comprising heart model setting means for constructing a heart model by establishing I, J, K blocks in respective vertical, horizontal, and height directions, and also by assigning cells to a desired one of the blocks, section display means for visually displaying a section of a desired one of the blocks of the heart model, operating means for assigning cells and types thereof to the block whose section is displayed by the section display means, cell type display means for displaying the cell types distinguishably according to the assigned types of the cells, and adjacent section display means for simultaneously displaying sections adjacent to the section which is displayed by the section display means and also a section perpendicular to the section which is displayed by the section display means.

The electrocardiographic process simulator, which obtains a vectorcardiogram through simulation, also comprises vectorcardiogram data storing means for storing data, from time to time, of the vectorcardiogram, display means for displaying the vectorcardiogram based on the data stored in the vectorcardiogram data storing means, display time designating means for designating a desired time contained in the data stored in the vectorcardiogram data storing means, and direction display means, responsive to an output signal from the display time designating means, for displaying a vectorcardiogram loop direction on the vectorcardiogram based on vectorcardiogram data at the designated time and vectorcardiogram data at a next time.

The above and further objects, details and advantages of the present invention will become apparent from the following detailed description of a preferred embodiment thereof, when read in conjunction with the accompanying drawings.

FIG. 1 is a block diagram of an electrocardiographic process simulator according to a preferred embodiment of the present invention;

FIG. 2 is a block diagram of a heart model setting means in the electrocardiographic process simulator shown in FIG. 1;

FIG. 3 is a flowchart of a processing sequence of the heart model setting means;

FIG. 4 is a diagram showing an image pattern displayed by a display means in the heart model setting means;

FIG. 5 is a graph showing an action potential waveform of a myocardial cell;

FIG. 6 is a perspective view showing the position of a heart model in a three-dimensional torso model;

FIG. 7 is a flowchart of a processing sequence of an excitation conduction processing means in the simulator;

FIG. 8 is a flowchart of one step of the processing sequence shown in FIG. 7;

FIG. 9 is a flowchart of a processing sequence of a body surface potential processing means in the simulator;

FIG. 10 is a diagram showing an image pattern displayed by the display means, the image pattern including 12-lead ECGs and VCGs which are displayed simultaneously;

FIG. 11 is a block diagram of a data output means for applying data to the display means;

FIG. 12 is a flowchart of a processing sequence of the data output means illustrated in FIG. 11;

FIGS. 13 and 14 are diagrams showing electrocardiographic and vectorcardiographic processes of a normal heart model and a heart model which suffers apical hypertrophic cardiomyopathy, which processes are simulated by the simulator of the present invention;

FIG. 15 is a diagram showing a 12-lead electrocardiogram which is clinically obtained from a heart with apical hypertrophic cardiomyopathy; and

FIG. 16 is a diagram showing body surface potential waveforms produced by the simulator of the present invention.

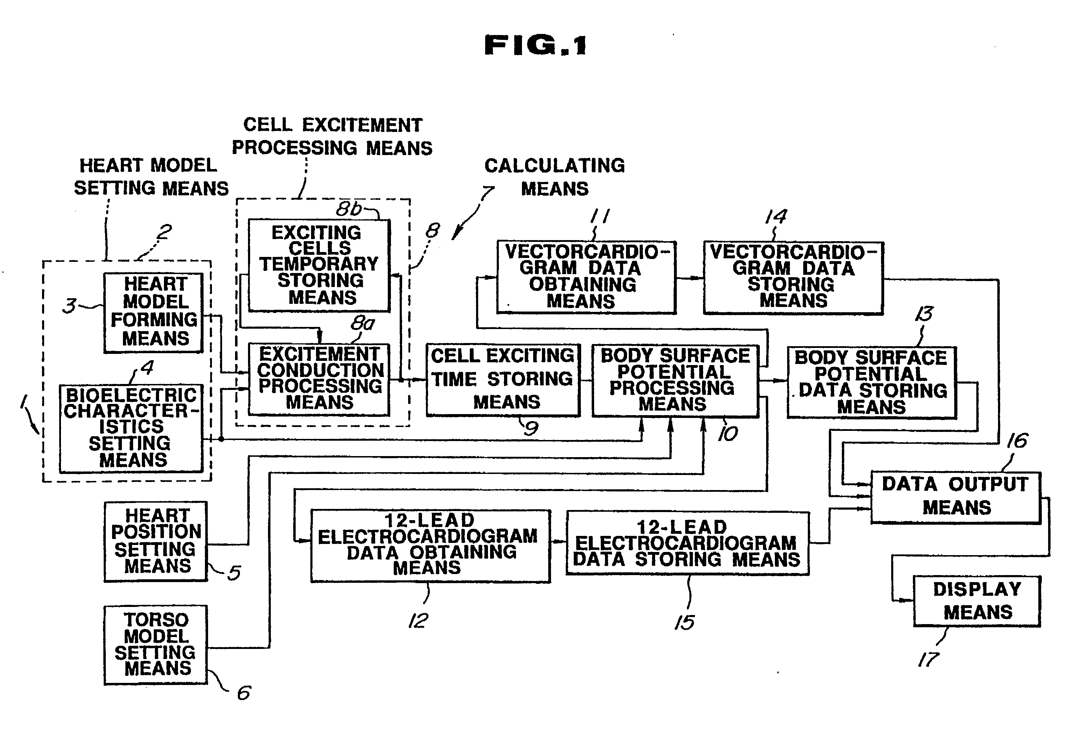

FIG. 1 shows in block form of an electrocardiographic process simulator according to the present invention. The simulator, generally denoted at 1, has a heart model setting means 2 which comprises a means 3 for constructing a three-dimensional heart model of various cells and a means 4 for setting bioelectric characteristics of the cells of the three-dimensional heart model which is constructed by the constructing means 3. The simulator 1 also has a means 5 for setting a position and an angle for the heart model, and a means 6 for setting a three-dimensional human torso model.

The simulator 1 further includes a calculating means 7 which has a cell excitation processing means 8, a cell exciting time storing means 9 for storing a cell exciting time which is calculated by the cell excitation processing means 8, a body surface potential processing means 10 for calculating potentials on the torso body surface based on calculated cell exciting time, a vectorcardiogram (VCG) data obtaining means 11 for obtaining VCG data, and a 12-lead electrocardiogram (ECG) data obtaining means 12 for obtaining 12-lead ECG data. The cell excitation processing means 8 comprises an excitation propagation or conduction processing means 8a and an excited-cell temporary storing means 8b. The data calculated by the body surface potential processing means 10 are sent to the VCG data obtaining means 11 and the 12-lead ECG data obtaining means 12. The data calculated by the body surface potential processing means 10, the VCG data obtaining means 11, and the ECG data obtaining means 12 are stored respectively by a body surface potential data storing means 13, a VCG data storing means 14, and a 12-lead ECG data storing means 15. Data to be supplied to a display means 17 are produced by a data output means 16 which are connected to the data storing means 13, 14, 15.

The heart model setting means 2 generates a three-dimensional heart model simulative of an actual human heart in a three-dimensional oblique coordinate system which has a vertical coordinate axis I, a horizontal coordinate axis J, and a height coordinate axis K along the sides of a parallelepiped. A myocardial cell of a desired type is assigned to a desired block of the heart model which is designated by coordinates (i, j, k) in the coordinate system.

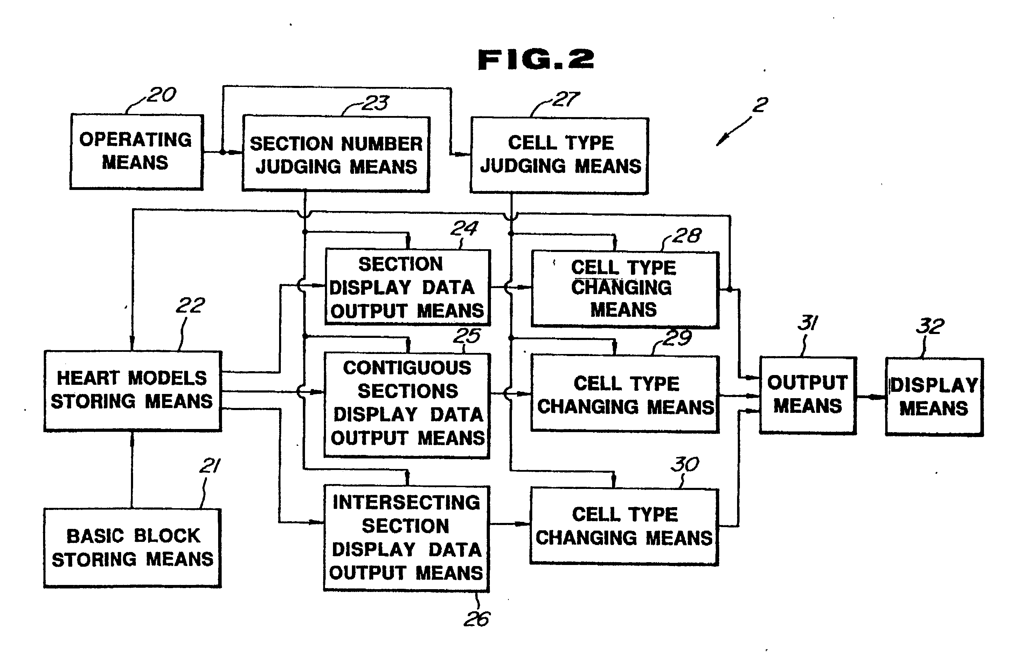

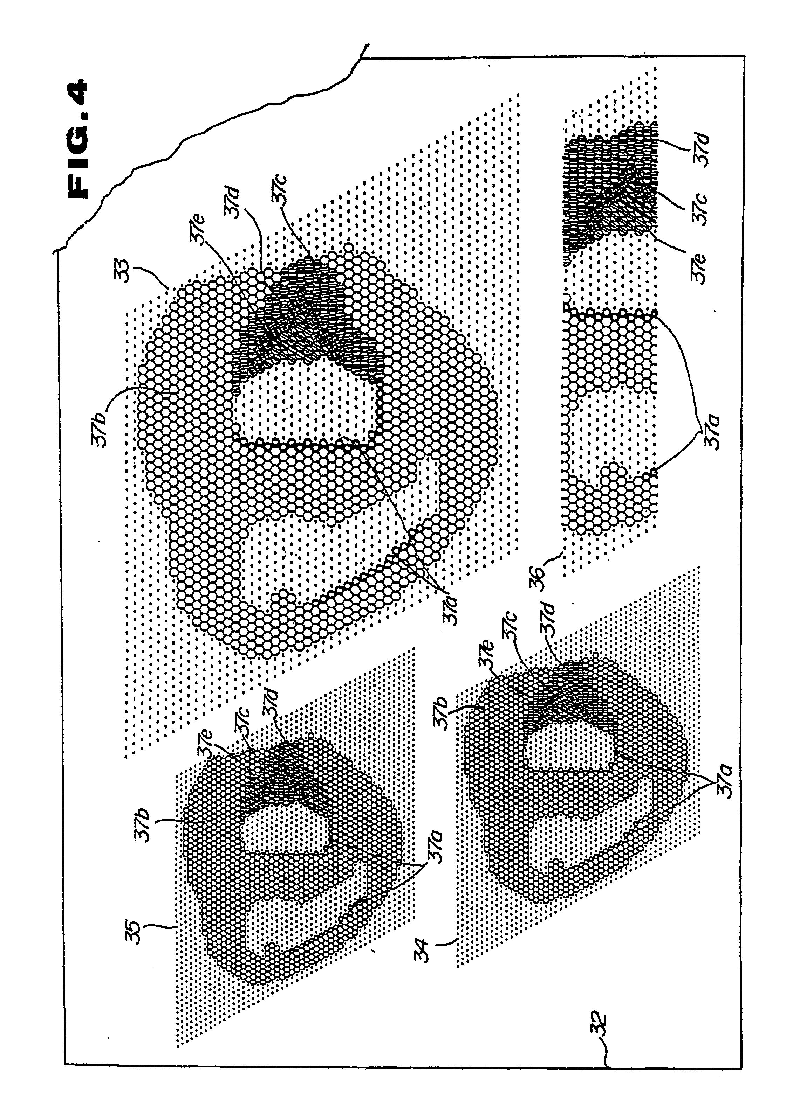

FIG. 2 shows the heart model setting means 2 in greater detail. The heart model setting means 2 includes an operating means 20 for designating a desired heart section and a myocardial cell type of a heart model which is to be modified or newly generated. Such a desired heart section can be designated by specifying a value on one of the coordinate axes I, J, K to select a desired plane in the coordinate system. For example, when a value K = ki is specified, a horizontal heart section as shown at an upper right corner in FIG. 4 is selected. A basic block storing means 21 serves to store I block coordinates along the vertical coordinate axis, J block coordinates along the horizontal coordinate axis, and K block coordinates along the height coordinate axis.

The heart model setting means 2 also has a heart model storing means 22 for storing setting data such as the coordinates of designated blocks and the types of myocardial cells assigned to the blocks, for a heart model which has been generated or is being generated. The heart model storing means 22 can store the data about a plurality of heart models which are given respective serial numbers. A section number judging means 23 judges a section number (= one coordinate value) which is designated by the operating means 20. In response to an output signal from the section number judging means 23, a section display data output means 24 selects designated section data from the data stored in the heart model storing means 22 and issues the selected section data. A contiguous section display data output means 25 similarly selects and issues the data of two sections which are adjacent to the above designated section, from the data stored in the heart model storing means 22. An intersecting section display data output means 26 also selects and issues the data of a section perpendicular to the designated section, from the data stored in the heart model storing means 22.

When a desired heart section is designated, two adjacent heart sections are automatically given, and a heart section perpendicular to the desired heart section can be specified as desired. For example, when a value K = ki is selected to designate a desired heart section, two heart sections indicated by the values K = ki + 1, K = ki - 1 are automatically given as adjacent sections, and a perpendicular heart section can be specified if a value on either the coordinate axis I or J is selected.

With the four heart sections being thus displayed, the cell type of a block is corrected or added by the operating means 20. In response to an output signal from the operating means 20, cell type changing means 28, 29, 30 change cell types in and/or add myocardial cells to the designated heart section which is being displayed, through a cell type judging means 27.

The types of myocardial cells represent the types of cells which make up a human heart, and include, for example, sinoatrial node, atrial muscle, atrioventricular node, bundle of His, bundle branches, Purkinje fibers, and ventricular muscle. Special cells which can be defined by the user of the simulator are also available. The electrophysiological characteristics of these cells are established by the electrophysiological characteristics setting means 4.

A heart section which is being set or to be corrected is displayed by a display means 32 through an output means 31.

When the above sequence of setting a heart model or correcting a heart model is finished, the set or corrected data are stored in the heart model storing means 22 again.

The display means 32 for displaying a heart model being set or corrected may double as the display means 17 shown in FIG. 1.

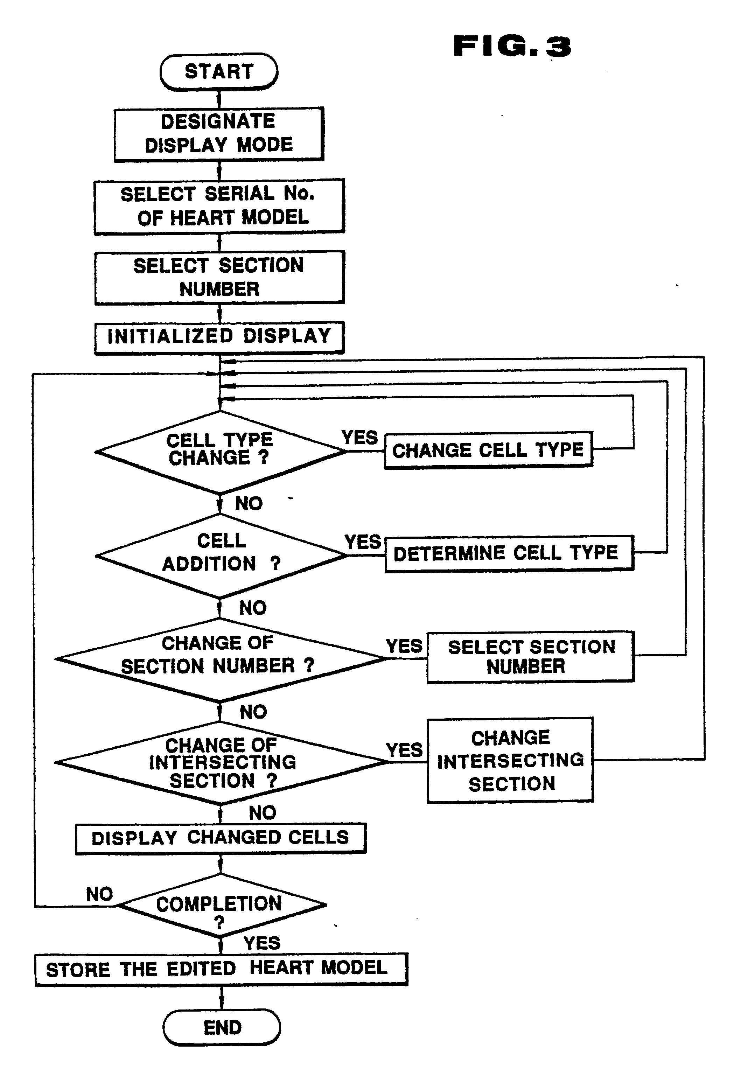

FIG. 3 shows a flowchart of a processing sequence of the heart model setting means 2.

FIG. 4 shows a display pattern displayed by the display means 32 while a heart model is being generated or corrected. A heart section 33 to be corrected is selected when a value K = ki is specified, the heart section 33 being in an I - J plane. Two heart sections 34, 35 are adjacent to the heart section 33, and are defined by the values K = ki + 1, K = ki - 1. A heart section 36 is a section perpendicular to the heart section 33 to be corrected, and can be designated when a value on the coordinate axis J is specified. The types of myocardial cells are denoted by 37a, 37b, 37c, 37d, 37e in each of the displayed sections. More specifically, those myocardial cells which are indicated by 37a are Purkinje fibers, those by 37b are atrial or ventricular muscle, and those by 37c, 37d, 37e are myocardial cells with different stages of ischemia.

The display pattern shown in FIG. 4 allows the user to visually perceive a three-dimensional heart model which is being generated or corrected, and at the same time those areas which surround the section being generated or corrected.

The bioelectric characteristics setting means 4 serves to set and store various parameters such as action potential, conduction speed, pacing time, for example, with respect to the various types of myocardial cells which are designated by the heart model forming means 3.

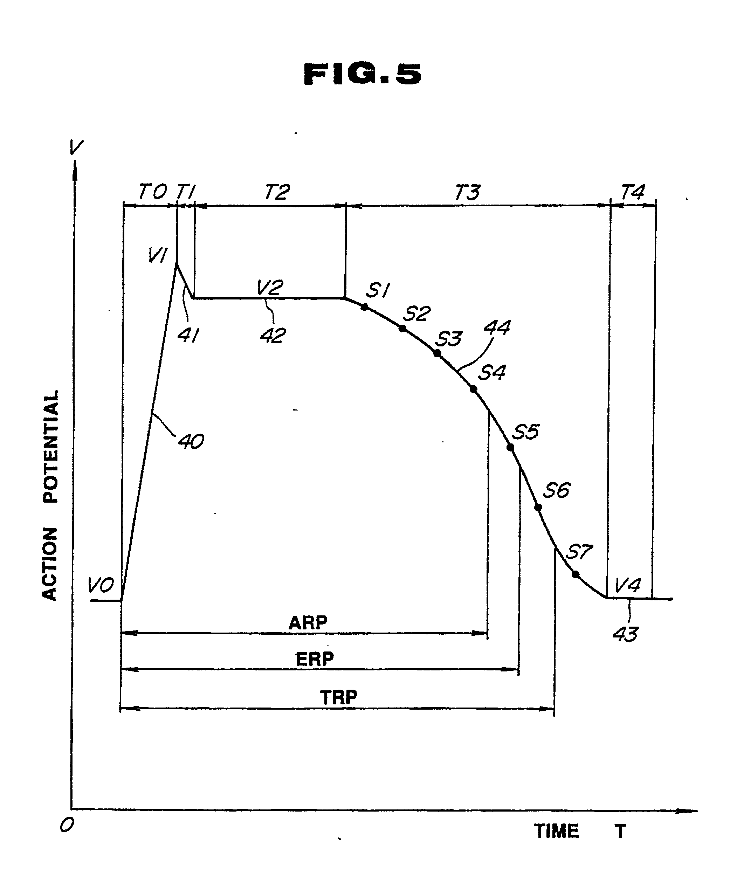

Table 1, given below, shows the types of such parameters. With the simulator of the present invention, it is possible to set and change 18 parameter types. FIG. 5 illustrates the correlation between parameters and an action potential waveform. To construct a normal heart model, the action potential waveforms of epicardial and endocardial cells are established such that they differ from each other only with respect to the time (ARP or T2) in which the action potential is sustained, i.e., the time in which the action potential is sustained for each cell is progressively or continuously longer as the cell is positioned farther from the epicardium and closer to the endocardium.

## Table 1

```
TO   : Interval of phase 0
T1   : Interval of phase 1
T2   : Interval of phase 2
T3   : Interval of phase 3
T4   : Interval of phase 4
V0   : Resting potential
V1   : Acting potential
V2   : Potential of phase 2
ARP  : Absolute refractory period
ERP  : Effective refractory period
TRP  : Total refractory period
SNP  : Supernormal period
TP   : Threshold potential
MDP  : Maximum diastolic potential
CS   : Conduction speed
GR   : Gradient of action potential distribution
PT   : Pacing time
CL   : Cycle length of pacing
```

As can be understood from Table 1 and FIG. 5, the action potential waveform in each of phase 0 (= depolarization) 40, phase 1 41, phase 2 (= plateau) 42, and phase 4 43 is defined by a straight line. The straight waveforms of the respective phases are determined by the times and potentials at the ends of the straight lines. The pericardial potential V (X) at the curve 44 in the phase 3 is given by the following Lagrange's interpolation polynominal according sample data Si (xi, yi) (S1 - S7):

$$V(X) = \sum_{k=1}^{n} Yk \left( \prod_{k \neq 1} \frac{X - Xi}{Xk - Xi} \right)$$

$$(n = 3)$$

In the simulator 1, the action potential waveform of each myocardial cell can be defined with few parameters. Therefore, the number of memories employed by the simulator 1 is reduced, and complex heart models can be constructed by the simulator 1.

The bioelectric characteristics setting means 4 cooperates with the heart model forming means 3 in freely establishing bioelectric characteristics such as the action potential waveform of each of the myocardial cells which make up a heart. Accordingly, the simulator 1 can generate a three-dimensional heart model close to an actual human heart, for simulation purpose.

Tables 2 and 3, given below, show examples of the values of the parameters with respect to Purkinje's network and ventricular muscle.

Table 2

| Cell type - Purkinge's network | |
|---|---|
| T0 : | 0 |
| T1 : | 0 |
| T2 : | 80 |
| T3 : | 175 |
| V0 : | -100.0 |
| V1 : | 20.0 |
| V2 : | 20.0 |
| ARP : | 200 |
| ERP : | 210 |
| TRP : | 230 |
| SNP : | 25 |
| TP : | .0 |
| MDP : | .0 |
| CS : | 5 |
| CD : Conduction direction | 1 |
| CL : (unit: 3ms) | 0 |
| F1 : Coefficient 1 | 5.0 |
| F2 : Coefficient 2 | .0 |
| F3 : Coefficient 3 | .0 |

Table 3

| Cell type - Ventricular muscle | |
|---|---|
| T0 : | 0 |
| T1 : | 0 |
| T2 : | 80 |
| T3 : | 175 |
| T4 : | 40 |
| V0 : | -100.0 |
| V1 : | 20.0 |
| V2 : | 20.0 |
| ARP : | 200 |
| ERP : | 210 |
| TRP : | 230 |
| SNP : | 25 |
| TP : | .0 |
| MDP : | .0 |
| CS : | 1 |
| CD : | 1 |
| CL : | 0 |
| F1 : | 5.0 |
| F2 : | .0 |
| F3 : | .0 |

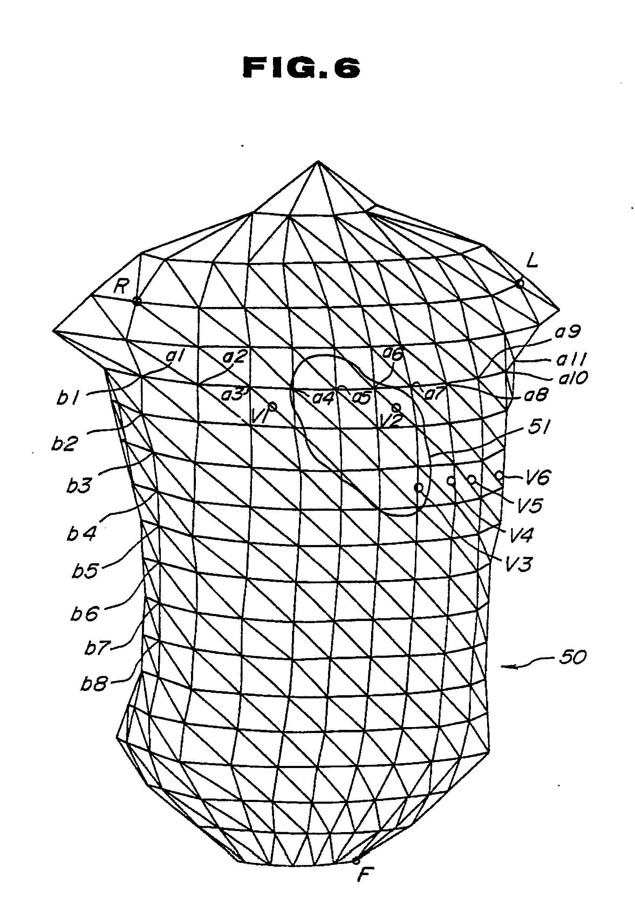

As shown in FIG. 6, the heart position setting means 5 and the torso model setting means 6 indicate the relationship between a torso model 50 and a heart model 51 positioned inside the torso model 50. The torso model set ting means 6 selects the size, shape, etc. of the torso model 50, whereas the heart position setting means 5 selects the position, angle, etc. of the heart model 51 with respect to the torso model 50. The positions of electrodes for generating a 12-lead ECG are denoted by R, L, F, V1 through V6.

In order to calculate a potential distribution which is developed on the surface of the body by an EMF distribution in the heart, the surface of the torso model 50 is covered with 344 nodes and 683 triangles connecting these nodes. The position of the three-dimensional heart model 51 in the torso model 50 is determined on the basis of a standard text on anatomy. Axis deviation can also be expressed easily. For the purpose of reducing the number of calculations needed, it is assumed that the electric conductivity in the body is uniform and that the boundary element method is used to calculate potentials, but it is possible to take into account nonuniformity introduced by a lung, if necessary. The potentials at the respective electrodes are calculated by way of linear interpolation from the potentials at the 344 nodes which are obtained by the boundary element method, and a 12-lead ECG and a body surface potential diagram are generated based on the calculated potentials. A VCG can be generated by combining current dipole moments of all the cells at the respective times and expressing the EMF in the heart with a single electric dipole.

The calculating means 7 for simulation will now be described below.

The excitation is propagated from cells to adjacent cells discretely, and the time required for the excitation to be conducted to an adjacent cell is referred to "1 STEP".

When a simulation process is started, the excitation conduction processing means 8a of the cell excitation processing means 8 determines cells which start automatic excitation, based on input data from the heart model forming means 3 and the bioelectric characteristics setting means 4. The coordinates of the excited cells thus determined are stored in the cell exciting time storing means 9 and the excited cell temporary storing means 8b.

Thereafter, each time 1 STEP elapses, there are determined cells which start being excited in response to the excitation conducted from the cells (the coordinates of which are stored in the temporary storing means 8b) excited in the previous STEP, and cells which newly start automatic excitation. The former cells can be determined from the cell coordinates established by the heart model forming means 3 and the conduction speed refractory period set by the bioelectric characteristics setting means 4, and also from the

7

previous exciting time stored in the cell exciting time storing means 9. The latter cells can be determined by searching for automatically excited cells established by the bioelectric characteristics setting means 4. The coordinates of the newly automtically excited cells and the cells which are excited in response to the conduction of excitation are stored in the storing means 9 and also in the storing means 8b again. Subsequently, the same operation is repeated for thereby simulating the process in which the excitation is conducted or propagated.

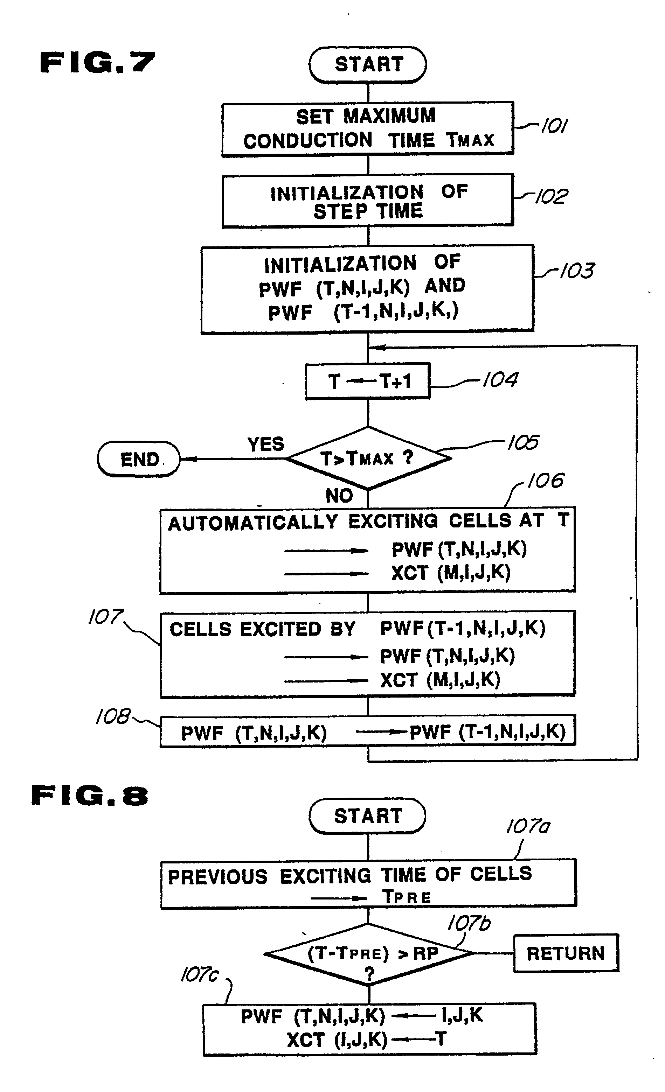

FIG. 7 is a flowchart of the above simulation process. The conducting time calculated by the simulator 1, which is 3 seconds in this embodiment, is set in a step 101. Initialization is effected in steps 102, 103. The STEP time is 3 msec. PWF indicates data stored in the excited cell temporary storing means 8b, and XCT indicates data stored in the cell exciting time storing means 9.

The STEP time is incremented one by one in a step 104. A next step 105 determines whether the incrementing the STEP time is finished or not. In a step 106, cells which are automatically excited at the time T are searched for from the group of myocardial cells which make up the three-dimensional heart model, and included in the PWF data and the XCT data. Cells which start being excited in response to the conduction of excitation are determined and included in the PWF data and the XCT data in a step 107. The PWF data are then replaced in a step 108.

FIG. 8 shows a detailed processing sequence of the step 107 which determines whether cells positioned in a conduction range are in a refractory period of not. Tpre in a step 107a indicates the previous exciting time of cells which are to be judged, and is stored in the cell exciting time storing means 9. RP in a step 107b represents the length of the refractory period, and is stored in the bioelectric characteristics storing means 4. Cells which are excited in response to the conduction of excitation are stored in a step 107c.

With respect to cells which start being excited in response to the conduction of excitation, the excitation conduction processing means 8a processes only those cells which surround cells that are excited at a predetermined time, i.e., only those cells to which it is possible for the excitation to be propagated. At the same time, the excitation conduction processing means 8a determines cells which are automatically excited. Therefore, those cells which start to be excited in a STEP next to a certain STEP can be determined in a minimum processing time.

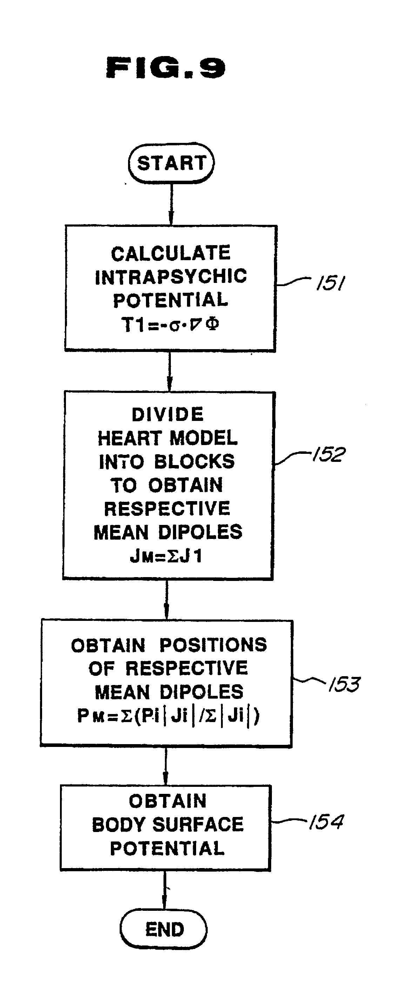

The boy surface potential processing means 10 will be described hereinbelow. FIG. 9 shows a flowchart of a processing sequence of the body surface potential processing means 10. In a step 151, an EMF distribution in the three-dimensional heart model is determined by determining a current dipole moment Ji for every other cell. The moment Ji of a certain cell is proportional to the spatial differential $\nabla\Phi$ of the pericardial potential $\Phi$ of the cell at the time, and is given by:

$$Ji = -\sigma \cdot \nabla\Phi$$

where $\sigma$ is the average electric conductivity of each myocar dial cell.

Then, the three-dimensional heart model is divided by m vertical planes, n horizontal planes, and k height planes into (m × n × k) blocks, and the mean dipole moments Jm of the respective blocks are determined in a step 152. The magnitude of the mean dipole moments Jm is determined as the sum of current dipole moments Ji of the cells contained in the blocks. A step 153 then determines the weighted mean Pm of the positions of the dipole moments Ji which are determined in the step 151. Pi in the step 153 indicates the position of an individual cell i. Based on all the mean dipole moments Jm thus determined, the body surface potential V at each electrode on the torso model 50 is determined in a step 153. If the surface of the torso model 50 is divided into n notes, the potential distribution V on the body surface is expressed as a n-dimensional vector, and given by:

$$V = V^{\infty} - A \cdot q^{\infty} - B \cdot \alpha^{\infty}$$

where $V^{\infty}$ and $q^{\infty}$ are the potential on the body surface in case there is a source distribution in an infinitely uniform medium and an N-dimensional vector indicative of normal differential of the potential, respectively, $\alpha$ is an (N + 1)-dimensional vector, and A and B are constants which are determined by the configuration of the body surface, the electric conductivity in the body, and the method of dividing the body surface.

According to the above process of calculating body surface potentials, the number of mean dipole moments Jm is much smaller than the number of electric dipole moments of all the cells. Consequently, the time required to calculate body surface potentials is much shorter as compared with the conventional process which directly calculates body surface potentials from the current dipole moments of all the cells. Furthermore, even if the heart model is divided into blocks at intervals each corresponding to 1/3 of the ventricles, the accuracy of calculations is subjected to only an error of 1 % or less.

In the above embodiment, the current dipole moment of every other cell is determined in view of the fact that the intrapsychic EMF distribution can be expressed as a continuous function. As a result, the calculation time is reduced to 1/8 of the time which is needed to calculate the current dipole moments of all the cells. In this case, any accuracy error is 1 % or smaller.

The body surface potential distribution data which are obtained in the above manner are stored in the body surface potential data storing means 13. Based on the body surface potential distribution data, the 12-lead ECG data obtaining means 12 calculates 12-lead ECG data and stores them in its storing means 15. The VCG data obtaining means 11 combines the dipole moments of the blocks thereby to calculate VCG data, and stores the VCG data in the storing means 14. The calculation of the above data with the means 10, 11, 12 is effected in each STEP from the start of excitation to the end of excitation.

The data output means 16 supplies the display means 17 with the data about a desired one of a VCG, a body surface potential distribution diagram, and a 12-lead ECG based on the data stored in the storing means 13, 14, 15.

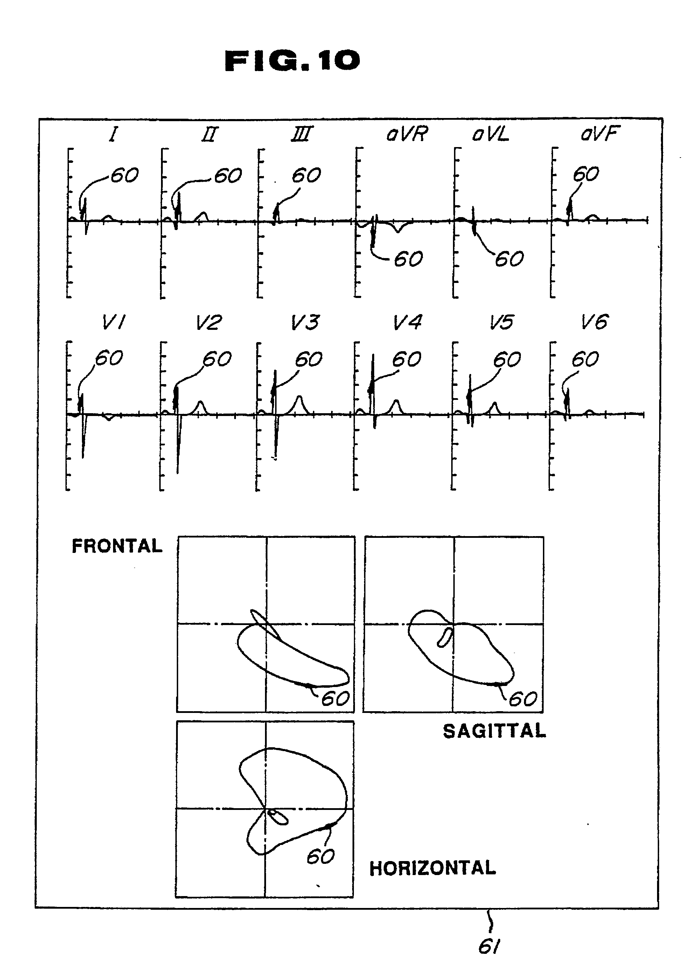

The data output means 16 enables the display means 17 to display VCGs and 12-lead ECGs on one screen 61 (FIG. 10) while also displaying the relationship in time therebetween. FIG. 10 shows 12-lead ECGs (I through V6) and VCGs (frontal, horizontal, and sagittal) which are displayed on the screen 61. Each of the displayed diagrams includes an arrow 60 whose starting end represents the data at a certain desired time, and whose direction represents the direction in which time goes on. If the desired time is changed and the starting end of the arrow 60 is changed, then the correlation with respect to time between the ECGs and the VCGs will be understood more clearly. The display pattern shown in FIG. 10 allows the user to easily recognize the correlation in time between the ECGs and the VCGs, and also to the direction of the loops in the VCGs at a desired STEP time. As a benefit, the VCGs can easily be understood, and hence the diagnosis of a heart disease can easily be performed.

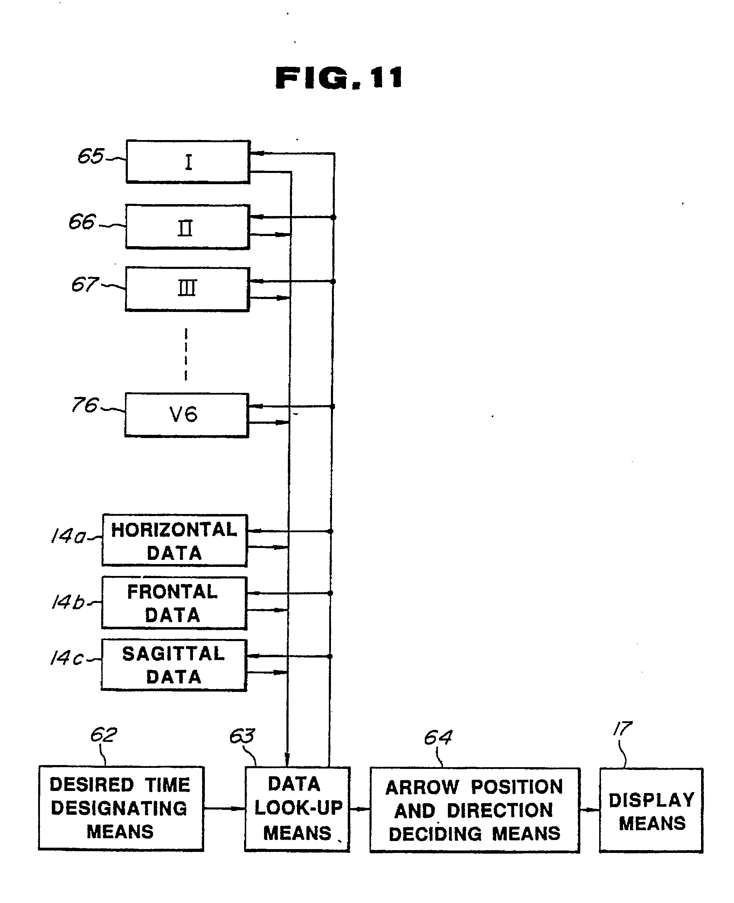

FIG. 11 shows an arrangement of the data output means 16 in specific detail. The data output means 16 has a desired time designating means 62 for designating the position of the arrow 60, i.e., a corresponding time, and a data look-up means 63 for searching for data at the designated time. The data look-up means 63 searches 12-lead ECG data storing means 65 through 76 and VCG data storing means 14a, 14b, 14c for desired data. The data output means 16 also has an arrow position and direction deciding means 64 for determining the displayed position and direction of the arrow 60 based on the data which are obtained.

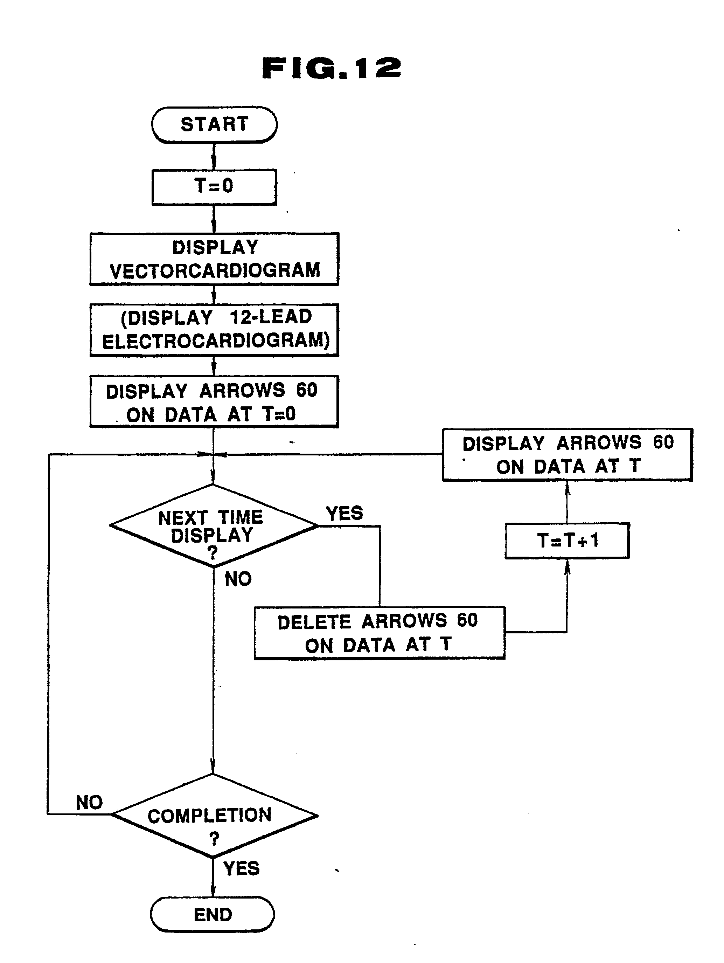

FIG. 12 is a flowchart of a processing sequence of the data output means 16.

Results of simulations of electrocardiographic processes effected by the simulator 1 will be described below.

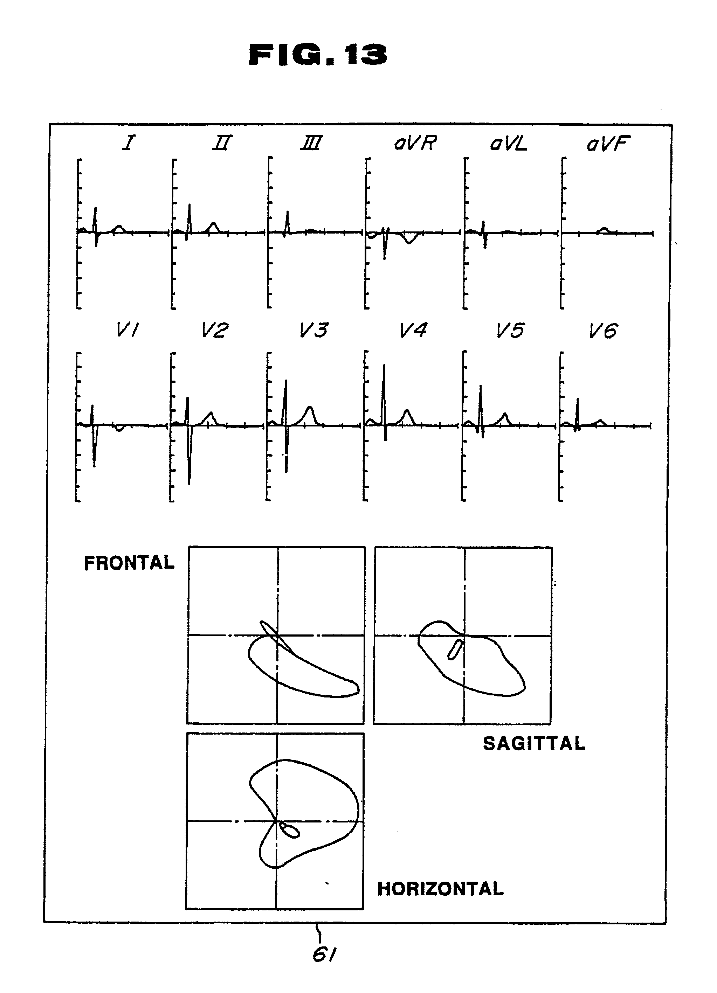

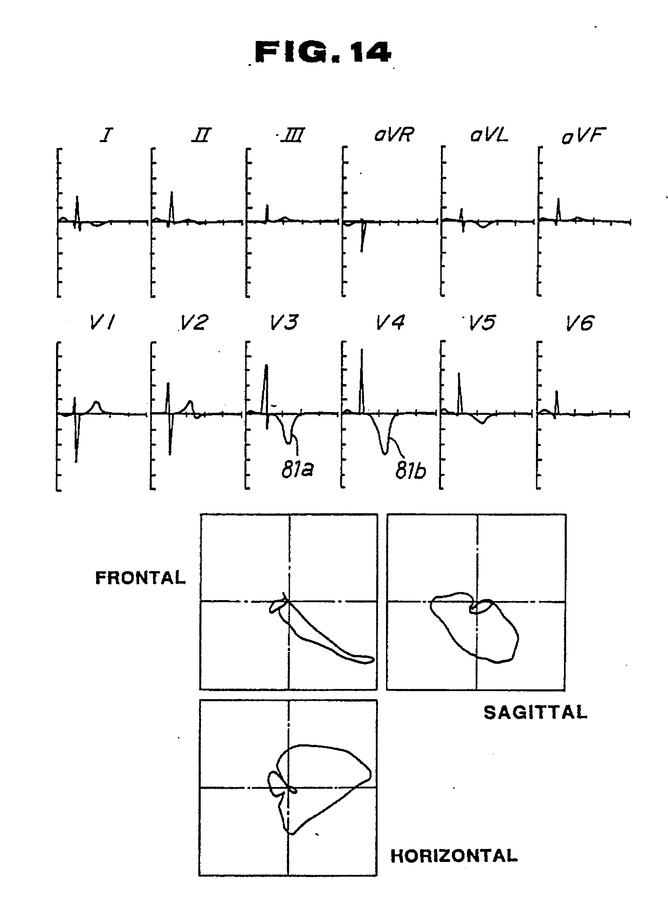

FIGS. 13 and 14 show 12-lead ECGs (I - V6) and VCGs of a normal heart model and a heart model with apical hypertrophic cardiomyopathy, which were simulated by the simulator 1. Comparison of these ECGs and VCGs indicates that the ECGs V3, V4 of the heart model with apical hypertrophic cardiomyopathy exhibit large negative T waves 81a, 81b.

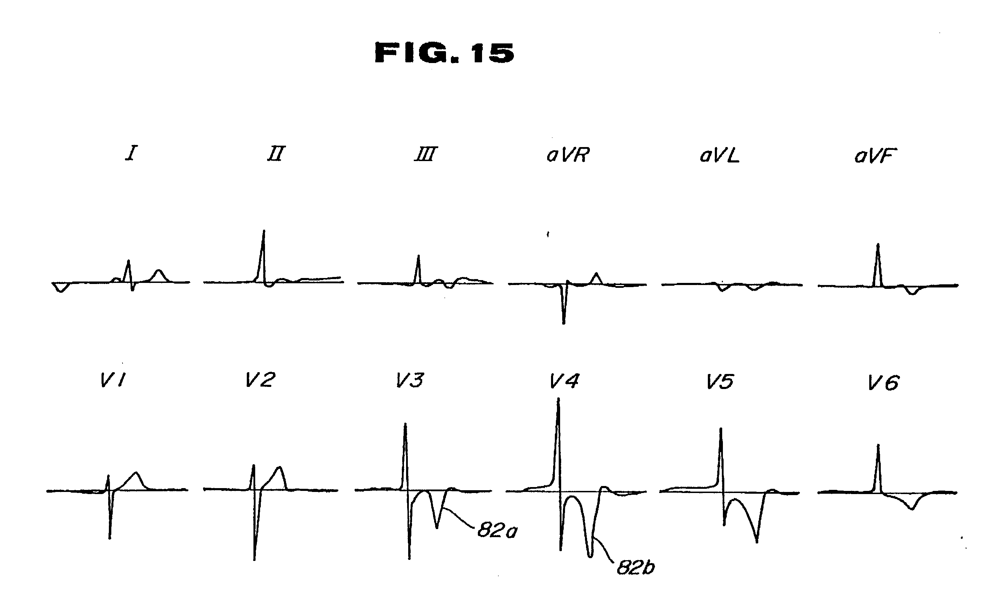

FIG. 15 shows 12-lead ECGs (I - V6) which were obtained through clinical measurements of a human heart with apical hypertrophic cardiomyopathy. The ECGs V3, V4 also indicate large negative T waves 82a, 82b. It can be seen from FIG. 15 that the simulator 1 is reliable in operation.

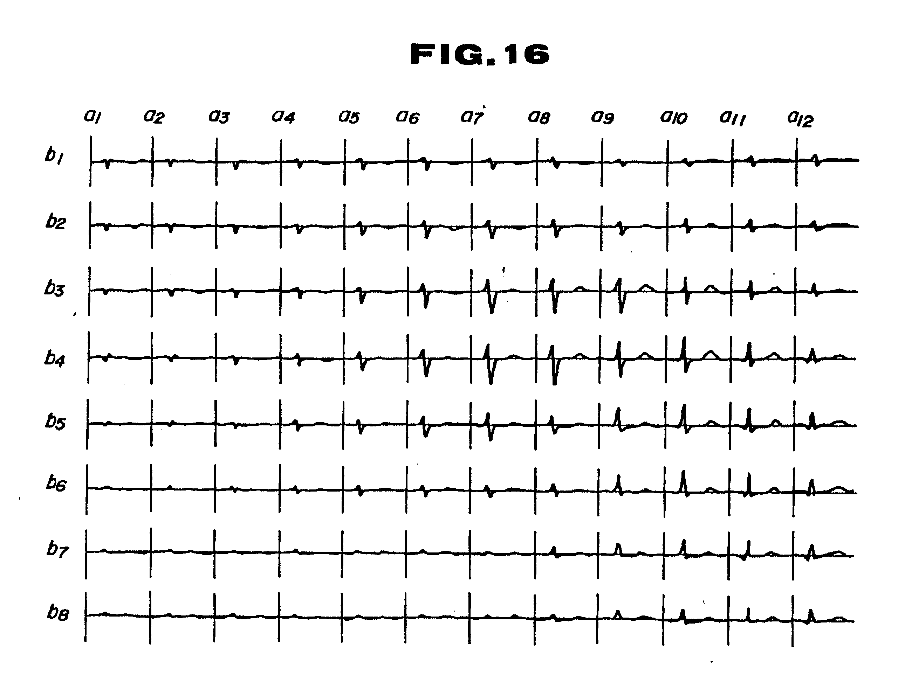

FIG. 16 illustrates simulated surface body potential waveforms together. Reference characters $a_1$ through $a_{12}$ indicate node numbers horizontally around the torso model 50 shown in FIG. 6, and reference characters $b_1$ through $b_5$ represent node numbers vertically along the torso model 50.

According to the simulator 1 of the present invention, it is possible to construct a three-dimensional heart model close to an actual human heart, and also to simulate a heart model with any desired heart disease, i.e., to obtain the correlation between an abnormal heart condition and body surface potentials, and to generate an ECG at any desired point on the body surface.

More specifically, since the simulator 1 of the present invention, unlike the conventional simulators, can establish desired bioelectric parameters with respect to each of the myocardial cells which make up the three-dimensional heart model 51, the whole processes of depolarization and repolarization of the ventricles can be simulated with respect to any desired heart disease. With respect to the three-dimensional heart model simulated by the simulator 1, not only the region and size of an abnormal heart disease, but also time-depending changes in the electrically excitation in myocardial cells, can freely be established. The simulator 1 is therefore effective for use in understanding basic medical knowledge and educational ECGs.

As shown in FIG. 4, when a three-dimensional heart model is generated or corrected on the simulator 1, a section 33 being generated or corrected, adjacent sections 3, 4, and a perpendicular section 5 are displayed for visual observation. Accordingly, the user can visually perceive immediately the relationship between an area being generated or corrected and surrounding areas, and also the relationship between such an area being generated or corrected and the entire heart model. Consequently, the processes of generating a heart model, adding cells, and changing cell types can easily be carried out.

Furthermore, cells which start being excited in a STEP next to a certain STEP can be determined in a

9

minimum processing time by the excitation conduction processing means 8a.

According to the simulator 1, body surface potentials are determined on the basis of the current dipole moment of every other cell, rather than all the cells which make up the heart model. Therefore, body surface potentials can be calculated within a minimum period of time while the desired calculation accuracy is being maintained.

Although there has been described what is at present considered to be the preferred embodiment of the present invention, it will be understood that the invention may be embodied in other specific forms without departing from the essential characteristics thereof. The present embodiment is therefore to be considered in all aspects as illustrative, and not restrictive. The scope of the invention is indicated by the appended claims rather than by the foregoing description.

## Claims

1. An electrocardographic process simulator for constructing a heart model of a cluster of cells and obtaining bioelectric phenomena of the heart model from the excited conditions of the cells which are determined by applying predetermined rules based on bioelectric characteristics of heart cells to the cells of the heart model, said electrocardiographic process simulator comprising :
heart model setting means (2) for constructing a heart model by establishing I, J, K blocks in respective vertical, horizontal, and height directions, and also by assigning cells to a desired one of said blocks ;
section display means (32) for visually displaying a section of a desired one of the blocks of said heart model ;
operating means (20) for assigning cells and types thereof to the block whose section is displayed by said section display means ;
cell type display means (32) for displaying the cell types distinguishably according to the assigned types of the cells ; and
adjacent section (33) display means (32) for simultaneously displaying sections (34, 35) adjacent to said section which is displayed by said section display means and also a section (36) perpendicular to said section which is displayed by said section display means.

2. An electrocardiographic process simulator according to claim 1, for obtaining a vectorcardiogram through simulation, comprising :
vectorcardiogram data storing means (14) for storing data, from time to time, of the vectorcardiogram ;
display means (17) for displaying the vectorcardiogram based on the data stored in said vectorcardiogram data storing means ; ·
display time designating means for designating a desired time contained in the data stored in said vectorcardiogram data storing means ; and
direction display means, responsive to an output signal from said display time designating means, for displaying a vectorcardiogram loop direction on said vectorcardiogram based on vectorcardiogram data at said designated time and vectorcardiogram data at a next time.

3. An electrocardiographic process simulator according to claim 1, for obtaining a vectorcardiogram and a 12-lead electrocardiogram through simulation comprising :
vectorcardiogram data storing means (14) for storing data, from time to time, of the vectorcardiogram ;
12-lead electrocardiogram data storing means (15) for storing data, from time to time, of the 12-lead electrocardiogram ;
display means (17) for simultaneously displaying the vectorcardiogram and the 12-lead electrocardiogram based on the data stored in said vectorcardiogram data storing means and the data stored in said 12-lead electrocardiogram data storing means ;
display time designating means for designating a desired time contained in the data stored in said storing means ; and
data display means (17) for displaying vectorcardiogram data and 12-lead electrocardiogram data corresponding to the designated time, on said vectorcardiogram and said 12-lead electrocardiogram which are displayed by said display means.

4. An electrocardiographic process simulator according to claim 1, wherein said heart model setting means comprises means for specifying the types of cells assigned to said desired block, said electrocardiographic process simulator further including :
bioelectric characteristics setting means (4) for giving the cells assigned by said heart model setting means parameters relative to bioelectric characteristics depending on the types of the cells.

5. An electrocardiographic process simulator according to claim 1, wherein said heart model setting means comprises means for designating desired ones out of the cells assigned to said desired block as cells which can automatically be excited, determining a pacing time for the cells, designating desired ones out of the remaining cells as cells which conduct excitation, determining a refractory period and a conduction time for the cells, and storing data with respect to said designated cells, said electrocardiographic process simulator further including :

cell exciting time storing means (9) for storing exciting times for all the cells which are available at all times from the start of simulation ;

excited cell temporary storing means (8b) for storing, at a desired step time, the cells which have been excited at a step time preceding said desired step time ; and

excitation conduction processing means (8a) for judging, at said desired step time, cells which can automatically be excited based on said pacing time, cells which have been excited at the preceding step time and which are stored in said excited cell temporary storing means, and cells which start being excited in response to the conduction of excitation based on a preceding cell exciting time stored in said cell exciting time storing means and said refractory period and said conduction time, and for storing again the cells excited at said step time as new data in said excited cell temporary storing means.

6. An electrocardiographic process simulator according to claim 1, further including :

torso model setting means (6) for setting and storing a torso model ;

heart position setting means (5) for selecting the position of the heart model with respect to the torso model ;

cell exciting time storing means (9) for storing exciting times for the respective cells at each step time, which times are calculated by a predetermined excitation conduction processing operation ;

bioelectric characteristics setting means (4) for storing active potential waveforms depending on the respective cells ;

current dipole moment calculating means (7) for calculating current dipole moments produced between a predetermined number of cells based on the exciting times for the cells which are stored in said cell exciting time storing means and the action potentials which are stored in said bioelectric characteristics setting means ;

mean dipole moment calculating means (7) for dividing said heart model into clusters of cells and calculating the size and position of one mean dipole moment from said current dipole moments for each of said clusters of cells ; and

body surface potential calculating means (7) for calculating body surface potentials from said mean dipole moment calculating means, said torso model, and the position of said heart model with respect to said torso model.

7. An electrocardiographic process simulator comprising :

heart model forming means (3) for giving desired spatial coordinates (i, j, k) to each of a plurality of hypothetical myocardial cells thereby to construct a hypothetical three-dimensional heart model simulative of an actual human heart ;

said hypothetical myocardial cells corresponding to a group of myocardial cells which have substantially uniform bioelectric characteristics ;

means (4) for establishing parameters (Table 1) which determine the bioelectric characteristics of each of said hypothetical myocardial cells, in a manner to simulate the actual human heart ;

means (7) for simulating a myocardial excitation process by starting automatic excitation of predetermined ones of said hypothetical myocardial cells and propagating the excitation to adjacent cells ; and

said parameter establishing means (4) comprising means for establishing times (ARP) in which action potentials are sustained, of said hypothetical myocardial cells of the three-dimensional heart model, such that said times are progressively longer from the epicardium toward the endocardium of the heart model.

8. An electrocardiographic process simulator according to claim 7, further including means (22) for storing said spatial coordinates (i, j, k) of said hypothetical myocardial cells of the three-dimensional heart model and said parameters (Table 1) which determine said bioelectric characteristics.

9. An electrocardiographic process simulator according to claim 7, wherein said heart model forming means (3) includes first display means (32) for displaying a desired section (33) of said three-dimensional heart model ;

said first display means (32) comprising means for displaying sections (34, 35) adjacent to said desired section (33) and a section (36) perpendicular to said desired section (33), simultaneously with said desired section (33).

10. An electrocardiographic process simulator according to claim 9, wherein said desired section (33) includes one of said hypothetical myocardial cells, with respect to which at least one of said spatial coordinates (i, j, k) and said parameters (Table 1) is to be established.

11. An electrocardiographic process simulator according to claim 7, further including second display means (17) for displaying results of the myocardial excitation process simulated by said simulating means (7).

12. An electrocardiographic process simulator according to claim 11, further including 12-lead electrocardiogram data obtaining means (12) for calculating 12-lead electrocardiogram data from the results of the myocardial excitation process simulated by said simulating means (7), vectorcardiogram data obtaining means (11) for calculating vectorcardiogram data from the results of the myocardial excitation process simulated by said simulating means (7), and body surface potential processing means (10) for calculating body surface potential distribution data from the results of the myocardial excitation process simulated by said simulating means (7) ;

said second display means (17) comprising means, responsive to the calculated data, for displaying at least one of a 12-lead electrocardiogram, a vectorcardiogram, and a body surface potential distribution.

13. An electrocardiographic process simulator according to claim 12, wherein said second display means (17) comprises means for displaying at least one of electrocardiograms of said 12-lead electrocardiogram simultaneously with the vectorcardiogram.

14. An electrocardiographic process simulator according to claim 13, wherein said second display means (17) comprises means for displaying data (60) corresponding to a desired time in the myocardial excitation process, on said vectorcardiogram and said 12-lead electrocardiogram which are simultaneously displayed.

15. An electrocardiographic process simulator according to claim 12, wherein said vectorcardiogram data obtaining means (11) for determining current dipole moments (Ji) of the respective hypothetical myocardial cells and combining said current dipole moments into a single dipole indicative of an electromotive force distribution in the heart model, thereby to obtain the vectorcardiogram data at respective times in the myocardial excitation process.

16. An electrocardiographic process simulator according to claim 12, further including means (6) for establishing a torso model (50) including said three-dimensional heart model ;

said body surface potential processing means (10) comprising means for dividing said three-dimensional heart model into a plurality of models, calculating mean dipole moments (Jm) with respect to the respective blocks from said hypothetical myocardial cells contained in said blocks, and calculating the body surface potential distribution data based on all the calculated mean dipole moments (Jm).

# FIG.1

EP 0 383 697 A2

# FIG.2

EP 0 383 697 A2

# FIG. 3

```
                    ( START )
                        │
            ┌───────────────────────┐
            │      DESIGNATE         │
            │    DISPLAY MODE        │
            └───────────────────────┘
                        │
            ┌───────────────────────┐
            │   SELECT SERIAL No.    │
            │   OF HEART MODEL       │
            └───────────────────────┘
                        │
            ┌───────────────────────┐
            │    SELECT SECTION      │
            │       NUMBER           │
            └───────────────────────┘
                        │
            ┌───────────────────────┐
            │  INITIALIZED DISPLAY   │
            └───────────────────────┘
```

CELL TYPE CHANGE ?  — YES →  CHANGE CELL TYPE

NO

CELL ADDITION ?  — YES →  DETERMINE CELL TYPE

NO

CHANGE OF SECTION NUMBER ?  — YES →  SELECT SECTION NUMBER

NO

CHANGE OF INTERSECTING SECTION ?  — YES →  CHANGE INTERSECTING SECTION

NO

DISPLAY CHANGED CELLS

COMPLETION ? — NO

YES

STORE THE EDITED HEART MODEL

( END )

FIG.4

EP 0 383 697 A2

EP 0 383 697 A2

# FIG.5

EP 0 383 697 A2

# FIG.6

**FIG.7**

```
            ( START )
               │
   ┌───────────────────────────┐
   │  SET MAXIMUM              │──── 101
   │  CONDUCTION TIME Tmax     │
   └───────────────────────────┘
               │
   ┌───────────────────────────┐
   │  INITIALIZATION  OF       │──── 102
   │  STEP TIME                │
   └───────────────────────────┘
               │
   ┌───────────────────────────┐
   │  INITIALIZATION  OF       │──── 103
   │  PWF (T,N,I,J,K) AND       │
   │  PWF (T-1,N,I,J,K,)        │
   └───────────────────────────┘
               │◄──────────────────────────┐
        ┌────────────┐                      │
        │ T ── T+1   │──── 104              │
        └────────────┘                      │
               │                            │
              ╱ ╲                           │
  YES        ╱   ╲  ──── 105                │
( END )◄────< T>Tmax? >                      │
             ╲   ╱                           │
              ╲ ╱                            │
               │ NO                          │
   ┌───────────────────────────┐            │
   │ AUTOMATICALLY EXCITING     │──── 106    │
   │ CELLS AT T                 │            │
   │    ──► PWF(T,N,I,J,K)       │            │
   │    ──► XCT (M,I,J,K)        │            │
   └───────────────────────────┘            │
               │                            │
   ┌───────────────────────────┐            │
107│ CELLS EXCITED BY  PWF(T-1,N,I,J,K)      │
   │    ──► PWF(T,N,I,J,K)       │            │
   │    ── XCT (M,I,J,K)         │            │
   └───────────────────────────┘            │
               │                            │
108┌───────────────────────────┐            │
   │ PWF (T,N,I,J,K) ──► PWF(T-1,N,I,J,K)    │
   └───────────────────────────┘────────────┘
```

**FIG.8**

```
            ( START )
               │
   ┌───────────────────────────┐
   │ PREVIOUS EXCITING TIME     │──── 107a
   │ OF CELLS ──► Tpre          │
   └───────────────────────────┘
               │
              ╱ ╲
             ╱   ╲  ──── 107b
            < (T-Tpre) > RP >──────► ( RETURN )
             ╲   ╱
              ╲ ╱ ?
               │
  107c┌───────────────────────────┐
      │ PWF (T,N,I,J,K) ◄── I,J,K  │
      │ XCT (I,J,K) ◄── T          │
      └───────────────────────────┘
```

# FIG.9

```
        ( START )
            │
            ▼
   ┌─────────────────┐
   │   CALCULATE     │
   │  INTRAPSYCHIC   │
   │   POTENTIAL     │──── 151
   │  T1=-σ·∇Φ       │
   └─────────────────┘
            │
            ▼
   ┌─────────────────┐
   │     DIVIDE      │
   │  HEART MODEL    │
   │  INTO BLOCKS    │
   │   TO OBTAIN     │──── 152
   │   RESPECTIVE    │
   │  MEAN DIPOLES   │
   │    JM=ΣJ1       │
   └─────────────────┘
            │
            ▼
   ┌─────────────────┐
   │ OBTAIN POSITIONS│
   │  OF RESPECTIVE  │
   │   MEAN DIPOLES  │──── 153
   │ PM=Σ(Pi│Ji│/Σ│Ji│)│
   └─────────────────┘
            │
            ▼
   ┌─────────────────┐
   │    OBTAIN       │
   │  BODY SURFACE   │──── 154
   │   POTENTIAL     │
   └─────────────────┘
            │
            ▼
        (  END  )
```

# FIG.10

# FIG. 11

EP 0 383 697 A2

# FIG.12

```
            ┌──────────┐
            │  START   │
            └────┬─────┘
                 │
            ┌────┴─────┐
            │   T=0    │
            └────┬─────┘
                 │
       ┌─────────┴──────────┐
       │      DISPLAY       │
       │ VECTORCARDIOGRAM   │
       └─────────┬──────────┘
                 │
       ┌─────────┴──────────┐
       │  (DISPLAY 12-LEAD  │
       │ ELECTROCARDIOGRAM) │
       └─────────┬──────────┘
                 │
       ┌─────────┴──────────┐
       │ DISPLAY ARROWS 60  │
       │  ON DATA AT T=0    │
       └─────────┬──────────┘
```

DISPLAY ARROWS 60
ON DATA AT T

NEXT TIME
DISPLAY
?

YES

NO

T=T+1

DELETE ARROWS 60
ON DATA AT T

NO

COMPLETION
?

YES

```
            ┌──────────┐
            │   END    │
            └──────────┘
```

EP 0 383 697 A2

# FIG. 13

61

EP 0 383 697 A2

# FIG. 14

I    II    III    aVR    aVL    aVF

VI    V2    V3    V4    V5    V6

81a    81b

FRONTAL

SAGITTAL

HORIZONTAL

# FIG. 15

I  II  III  aVR  aVL  aVF

V1  V2  V3  V4  V5  V6

82a

82b

EP 0 383 697 A2

EP 0 383 697 A2

## FIG.16